# FASCICULE DE BREVET EUROPEEN

(11) **EP 1 519 943 B1**
(45) Date de publication et mention de la délivrance du brevet: **28.04.2010**
(21) Numéro de dépôt: 03763949.9
(22) Date de dépôt: 09.07.2003
(51) Int. Cl.: A61K 31/57, C07J 41/00, A61P 15/04, A61P 15/06, A61P 15/12, A61P 9/12

(54) **COMPOSES A BASE D'HORMONE DONNEURS DE MONOXYDE D'AZOTE ET LEUR UTILISATION EN OBSTETRIQUE ET EN GYNECOLOGIE**
STICKSTOFFMONOXID-DONORVERBINDUNGEN AUF HORMONGRUNDLAGE UND DEREN VERWENDUNG IN DER GEBURTSHILFE UND GYNÄKOLOGIE
HORMONE-BASED NITROGEN MONOXIDE DONOR COMPOUNDS AND USE THEREOF IN OBSTETRICS AND GYNECOLOGY

(30) Priorité: 09.07.2002 FR 0208619
(43) Date de publication de la demande: 06.04.2005
(73) Titulaire: Effik, 91572 Bievres (FR)
(72) Inventeur: GRASSET, Etienne, Alfred, Robert, F-92100 Boulogne-Billancourt (FR); MENDEZ, Félix, E-28028 Madrid (ES); PAVAN, Carlo, F-94130 Nogent sur Marne (FR); TERRACOL, Didier, F-91370 Verrières le Buisson (FR)
(74) Mandataire: Casalonga, Axel
(86) Numéro de dépôt international: PCT/FR2003/002152
(87) Numéro de publication internationale: WO 2004/007522

(56) Documents cités:
- WO-A-98/09948
- DE-A- 1 643 034
- GB-A- 1 446 126
- US-A- 3 052 675
- US-A- 3 352 891
- US-A- 3 494 941

## Description

La présente invention concerne des composés capables de se fixer à des récepteurs d'hormones et présentant un groupement ester nitrique donneur de monoxyde d'azote, leur utilisation pour prévenir des naissances avant terme, augmenter la dilatation du col de l'utérus à terme, dans le cadre d'une thérapie substitutive hormonale ou en tant qu'anti-hypertenseur et leur application thérapeutique pour la préparation d'un médicament utilisable en obstétrique et en gynécologie.

Les naissances avant terme demeurent relativement communes, représentant de 5 à 10 % de toutes les grossesses. Cette proportion n'a pas évolué dans les 20-30 dernières années.

Une sécrétion adéquate de progestérone est en fait nécessaire au maintien de la grossesse jusqu'à terme. La progestérone est une hormone initialement secrétée par le corpus luteum ou corps jaune dans l'ovaire puis par le placenta. L'inhibition de la contractilité utérine par la progestérone ou les dérivés de la progestérone a été démontrée en utilisant des modèles animaux pharmacologiques.

L'administration de progestérone durant le troisième trimestre de la grossesse afin de prévenir une délivrance avant la maturité de l'enfant a déjà été évoquée il y a plus de 20 ans. De ce fait, la progestérone micronisée a été administrée de façon très commune en France à des doses orales élevées (le plus souvent au moins 600 mg par jour) à des femmes afin d'éviter un accouchement prématuré. Cette utilisation a cependant été arrêtée à la suite d'un risque de toxicité hépatique lié au traitement. De plus, ce traitement n'avait pas en fait d'incidence vraiment prouvée sur le poids de naissance et l'age de gestation des enfants.

Il a été montré très récemment qu'un traitement par injection hebdomadaire d'un dérivé de la progestérone permettait, chez des femmes à très haut risque, de réduire la fréquence des accouchements prématurés et de diminuer la fréquence de certaines complications chez leurs enfants *[*Meis PJ, Klebanoff M, Thom E et Coll. Prevention of recurrent preterm delivery by 17 alpha-hydroxyprogesterone caproate. N Engl J Med 2003;348:2379-85*]*

Le monoxyde d'azote a récemment été identifié comme étant un modulateur important de la fonction cellulaire. Il a un effet relaxant puissant sur les cellules du muscle lisse. En fait, le trinitrate de glycérile était utilisé depuis de nombreuses années pour soigner les symptômes d'angine de poitrine bien avant qu'on connaisse son mécanisme d'action (le trinitrate de glycérile est un donneur de monoxyde d'azote).

Le muscle de l'utérus, le myomètre, contient également des cellules musculaires lisses. La monoxyde d'azote synthase, l'enzyme qui synthétise le monoxyde d'azote, a ainsi un effet relaxant sur les cellules musculaires du myomètre de l'utérus et du col de l'utérus. Le monoxyde d'azote augmente également le flux sanguin utérin pendant la grossesse.

*In vivo,* l'inhibition de la production de monoxyde d'azote provoque une parturition avant terme chez les cobayes gestantes. Inversement, l'application locale à terme sur le col de l'utérus de nitroprusside de sodium, qui est un donneur de monoxyde d'azote, augmente la dilatation cervicale. Ceci montre que le monoxyde d'azote peut avoir deux effets différents, au niveau du corps de l'utérus, et au niveau du col. L'inhibition de la contraction de l'utérus relaxe le corps de l'utérus et peut prévenir un accouchement prématuré, avant terme. Lorsque le terme est atteint, l'induction d'un ramollissement et d'une dilatation du col peut aider à la progression de l'accouchement.

Les groupements donneurs d'acide nitrique tels que le trinitrate de glycérile ont ainsi été évalués chez la femme pour tester l'induction de la relaxation utérine. Des patientes dont l'état nécessitait une tocolyse urgente pour rétention placentaire, pour des problèmes d'extraction foetale difficile ou pour inversion utérine aiguë ont été traitées avec une dose initiale intraveineuse de 50 à 500 microgrammes de nitroglycérine avec des bolus répétés à intervalles de 60 à 90 secondes.

Malgré l'efficacité de ce traitement, on a noté dans 41 % des cas la survenue d'une hypotension transitoire en dépit d'une hydratation intraveineuse *[*O'Grady JP, Parker RK, Patel SS. Nitroglycerin for rapid tocolysis: development of a protocol and a literature review. J Perinataloy 2000;1:27-33*].*

L'administration de nitroglycérine grâce à un timbre transdermique capable de délivrer 0,4 mg/heure de nitroglycérine a été testée sur un total de 33 femmes présentant un travail avant terme et comparée à un placebo. Ces femmes ont reçu une administration prophylactique en intraveineuse de 1000 ml d'une solution saline à 0,9 % pendant 1 à 2 heures pour éviter l'hypotension induite par la nitroglycérine. Les femmes qui ont reçu la nitroglycérine ont moins d'accouchement dans les 48 heures. Cependant, l'effectif était trop faible pour être interprétable au niveau statistique.

Une étude plus importante a comparé l'efficacité du trinitrate de glycérile transdermique par des timbres de 10 ou 20 mg à une injection de ritodrine en intraveineuse afin d'éviter une délivrance avant terme. Les résultats obtenus montrent que l'efficacité de ces deux traitements est similaire *[*Lees CC, Lojacono A, Thompson C, Danti L, Black RS, Tanzi P et al. Glyceryl trinitrate and ritodrine in tocolysis: an international multicenter randomized study. Obstet Gynecol 1999;94:403-8*].* L'effet indésirable le plus courant observé avec la ritodrine était une tachycardie; pour le trinitrate de glycérile, c'étaient les maux de tête.

Le brevet WO 95 13802 décrit l'utilisation d'un substrat de la monoxyde d'azote synthase et/ou d'un donneur de monoxyde d'azote avec différents composés dont un progestatif afin d'inhiber la contraction utérine ou un inhibiteur de monoxyde d'azote avec un antagoniste de la progestérone afin de stimuler la contractilité utérine.

Le brevet US 5 895 783 décrit l'administration simultanée de trois composés, un progestatif, un substrat de la synthase d'acide nitrique ou un donneur d'oxyde nitrique et différents composés à vocation anti-inflammatoire tels que les inhibiteurs de cyclo-oxygénase chez des femmes présentant une éclampsie avec ou sans travail avant terme.

La nitroglycérine, l'arginine et la progestérone sont administrées dans des compositions séparées. Mais le dosage de progestérone doit être très élevé pour équivaloir à une quantité de 50 à 300 mg de progestérone injectée.

Le brevet US 5 595 970 décrit l'utilisation d'un substrat de monoxyde d'azote et/ou d'un donneur d'oxyde nitrique seul ou en combinaison avec un progestatif, un oestrogène ou les deux pour tempérer la survenue des symptômes liés à la ménopause. Dans ce cas, la quantité de progestatif est équivalente à une dose de 50 à 300 mg de progestérone injectée, ce qui est extrêmement élevé.

Le brevet US 6 040 340 décrit une méthode pour améliorer le taux de grossesse par administration d'un substrat de monoxyde d'azote et/ou d'un donneur d'oxyde nitrique seul ou en combinaison avec un progestatif et de façon optionnelle, avec un oestrogène ou une méthode contraceptive. Là encore, une dose très élevée de progestatif a été utilisée.

Le brevet US 6 028 106 décrit une méthode similaire pour relaxer le détrusor vésical et traiter l'incontinence urinaire.

Le brevet WO 98 09948 décrit l'utilisation de composés dérivés de l'oxyde nitrique qui peuvent relaxer le muscle lisse et aider à contrôle l'incontinence urinaire et d'autres pathologies qui incluent la délivrance avant terme. Une combinaison d'un groupement capable de relarguer du monoxyde d'azote avec des corticostéroïdes a été considérée seulement dans le but d'augmenter les propriétés anti-inflammatoires de ces corticoïde.

Le document DE 16 43 034 A (SCHERING AG) divulgue un procédé pour la préparation de dérivés esters nitriques tertiaires ou secondaires de stéroide alcool. Les médicaments revendiqués sont capables de se fixer à des récepteurs d'hormones et porteurs de groupement NO₂.

La demanderesse a découvert une famille de composés capables de se fixer à des récepteurs d'hormones et comportant un groupement ester nitrique donneur de monoxyde d'azote. Elle a découvert que ces composés exerçaient une action thérapeutique dans la prévention de naissances prématurées, dans le contrôle de la motilité utérine, dans l'augmentation de la dilatation cervicale lors de l'étape du travail, dans les anomalies de la contraction utérine, dans les dysménorrhées, dans le traitement hormonal de substitution des femmes ménopausées, dans l'induction d'un cycle endométrial dans le cadre de la reproduction assistée et dans la prévention de l'hypertension.

L'invention a donc pour objet des hormones capables de se fixer à des récepteurs d'hormones choisies parmi la progestérone et la 17OH progestérone, comportant un ou plusieurs groupements NO2 ayant au moins une des deux caractéristiques suivantes :
a) le groupe NO2 est lié directement à l'hormone par l'intermédiaire de l'atome d'oxygène de la forme énolique de l'hormone ;.
b) le groupement NO2 est lié par l'intermédiaire d'un radical benzyloxy à l'hormone, soit directement à l'hormone, soit par l'intermédiaire d'un groupement établissant un pont, tel qu'un groupement C=O ou COO, à l'atome d'oxygène de la forme énolique de l'hormone ou directement sur le cycle.

Les récepteurs d'hormone sont choisis parmi des récepteurs stéroïdes, rétinoïdes et de dérivés de la vitamine D.

Les composés selon l'invention se fixent aux tissus riches en récepteur hormonal. La présence de ces groupements au sein de ces molécules offre plusieurs avantages:
- modifications de propriétés physicochimiques de ces molécules avec, par exemple, amélioration de la solubilité dans l'eau des molécules les moins polaires,
- modification du métabolisme de ces molécules.
- possibilité délivrance du monoxyde d'azote an sein des tissus.

Ces hormones comportent au moins un ou plusieurs groupements ester nitrique donneur de NO.

L'hormone capable de se fixer à des récepteurs d'hormones est la progestérone ou la 17OH progestérone comportant un ou plusieurs groupements NO₂ lié (s) directement ou par l'intermédiaire d'un radical benzyloxy.

En particulier, lorsque le ou les groupements NO₂ sont liés directement à l'hormone, la liaison se fait par l'atome d'oxygène de la forme énolique de celle-ci par exemple en position 3 de la progestérone ou en position 17 de la 17OH progestérone.

Lorsque le ou les groupements NO₂ sont liés par l'intermédiaire d'un radical benzyloxy à l'hormone, cette liaison se fait soit directement à l'hormone, soit par l'intermédiaire d'un groupement établissant un pont, tel qu'un groupement C=O ou COO, à l'atome d'oxygène de la forme énolique de l'hormone ou directement sur le cycle tel que par exemple en position 3 de la progestérone ou en position 3 et/ou 17 de la 17OH progestérone.

Ces hormones peuvent être de formule suivante :

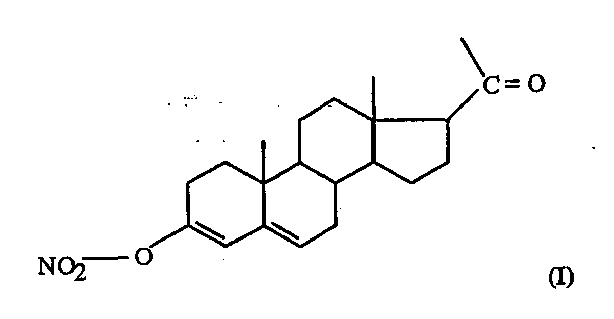

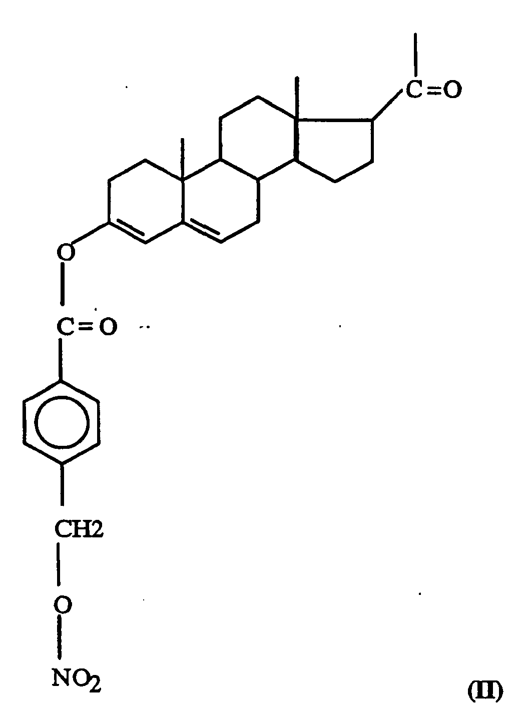

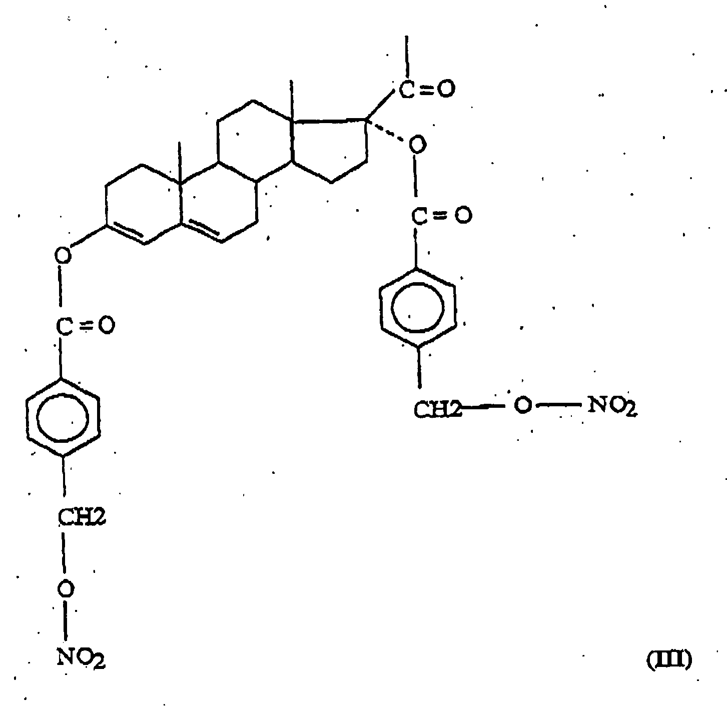

Il a été découvert que les composés de la présente invention définis ci-dessus sont utilisables dans la prévention des naissances avant terme, dans le contrôle de la motilité utérine et dans l'augmentation de la dilation du col de l'utérus au moment du travail. Ces composés sont également utilisables dans le traitement des anomalies de la contraction utérine et les dysménorrhées.

Les composés selon l'invention sont également intéressants dans le cadre d'une thérapie substitutive pour les femmes ménopausées, pour l'induction d'un cycle artificiel dans la procréation assistée et dans le cadre d'un usage en tant qu'antihypertenseur.

Ces propriétés justifient leur application en thérapeutique et l'invention a particulièrement pour objet à titre de médicaments, les composés tels que définis ci-dessus dans un milieu pharmaceutiquement acceptable.

Les composés conformes à l'invention présentent par rapport aux hormones stéroïdes non substituées en particulier les avantages suivants : une modification de leurs propriétés physico-chimiques, avec dissolution plus facile et régulière, meilleure solubilité,
- une action pharmacologique plus régulière, moins variable dans le temps et selon les individus
- l'obtention d'une accumulation sélective et/ou une délivrance de monoxyde d'azote dans les tissus qui possèdent des récepteurs stéroïdes
- une augmentation de l'activité due à l'activité synergique avec la molécule stéroïde au sein du même tissu ;

Les différentes formes pharmaceutiques et les voies d'administration peuvent être :
- pour un usage oromuqueux : des comprimés buccaux ou oromuqueux ou de tels comprimés à effet retard, une pâte ou un gel gingival, une tablette à mâcher, une gomme à mâcher, une pastille, une capsule oromuqueuse, des gouttes, gel, un spray en pâte ou n'importe quelle forme d'administration adéquate,
- pour un usage oral : des cachets, capsules, tablettes, tablettes recouvertes, capsules gastrorésistantes ou granulés,
- pour un usage nasal : un spray nasal, une solution à inhalation nasale, des gouttes nasales ou n'importe quelle autre forme d'administration nasale,
- pour une utilisation vaginale : un gel endocervical, une poudre endocervicale, une poudre pour un gel endocervical, une tablette servant à une solution vaginale, une émulsion vaginale, une capsule vaginale, une crème vaginale, une mousse vaginale, un gel vaginal, une irrigation vaginale, une solution vaginale ou une suspension vaginale, une tablette vaginale, un tampon vaginal ou n'importe quel autre moyen pouvant être retiré pour une administration vaginale ou cervicale (tel qu'un ruban imprégné),
- pour un usage sous-cutané : une administration intramusculaire ou sous-cutanée (incluant une infusion continue à l'aide d'une pompe) ou une administration intraveineuse telle qu'une solution, suspension ou émulsion,
- pour une utilisation topique ou transdermale : un timbre, une crème, poudre ou spray.

Les exemples suivants illustrent l'invention sans toutefois la limiter :

### Exemple 1 : préparation de l'ester nitrique d'énol 3 pregna-3(4)5(6)diène-20one

La progestérone (une part de progestérone en poids) est dissoute dans un mélange de 5 volumes d'acétonitrile et de 2 volumes de tétrahydrofurane (THF) maintenu à 0°. Quand la réaction est complète, une solution d'acide paratoluène sulfonique à raison de 0,025 part dans 0,25 volume d'acétonitrile est ajoutée. Une solution d'acide nitrique à 65 % (1,1 mole d'acide nitrique par mole de progestérone) dissoute dans 2 volumes d'acétonitrile est alors ajoutée. Après un temps de réaction d'approximativement 30 minutes, le produit est extrait avec le THF, la solution est concentrée par évaporation et cristallisée dans l'éther isopropylique.

On obtient le nitrate d'énol de progestérone de formule (I).

### Exemple 2 préparation d'un dérivé nitrique de progestérone de formule (II)

La progestérone à raison d'l part en poids est dissoute dans un mélange de 5 volumes d' acétonitrile et de 2 volumes de THF maintenu à 0°. Quand la réaction est complète, une solution d'acide paratoluène sulfonique à raison de 0,025 part dans 0,25 volume d'acétonitrile est ajoutée. Une solution de 4-nitroxy méthylbenzoate d'éthyle (1,1 mole par mole de progestérone) dissoute dans 2 volumes d'acétonitrile est ajoutée. Après un temps de réaction d'approximativement 30 minutes, le produit est extrait avec le THF, la solution est alors concentrée par évaporation et cristallisée dans l'isopropyléther.

### Exemple 3 : préparation de l'ester dinitrique de la 17 α-hydroxyprogestérone de formule (III)

La 17α-hydroxyprogestéroné à raison d'une part en poids est dissoute dans 2,25 volumes d'anhydride acétique (pur à au moins 98 %) maintenue à 0°C. Une solution de 2,5 % d'acide paratoluène sulfonique maintenue à 0 C dans 0,25 volume d'anhydride acétique est alors ajoutée. Le mélange est chauffé avec précaution à une température entre 0 et 20°. Une solution à 65 % de 4-nitroxyméthyl benzoate d'éthyle (1,1 mole par mole) dans 2 volumes d'acétonitrile est ajoutée. Le mélange est maintenu pendant une heure à 20° puis 0,05 part en poids de triéthylamine est ajoutée et le produit est mélangé à 10 parts d'un mélange eau + glace. Le produit est filtré et lavé puis resolubilisé dans du méthanol qui contient 10 % d'eau, acidifié avec de l'acide nitrique (0,9 mole par mole) et chauffé à reflux pendant 2 heures. Trois volumes de chlorure de méthylène sont ensuite ajoutés. Le produit est neutralisé, refroidi et extrait avec du chlorure de méthylène. Le chlorure de méthylène est alors éliminé et le produit est cristallisé dans du méthanol.

### Exemple 4: Inhibition in vitro de la contractilité utérine de fibres de myomètre d'utérus de femmes non gestantes

L'activité contractile de fragments de myomètres humains a été étudiée *in vitro.*

Après un temps de latence d'environ deux heures, permettant l'établissement de contraction spontanées, l'inhibition des contractions produite par l'addition dans le milieu de 17-α hydroxyprogestérone et de son dérivé nitré, (4'-nitro.oxy-méthyl)-benzoate de 17α hydroxy-pregn-4-éne-3,20-dione, identifié dans ce tableau sous le nom de (4'-nitro.oxy-méthyl)-benzoate de 17α hydroxy-pregn-4-ène-3,20-dione (composé 4) a pu être mesurée.

Cette expérience permet d'évaluer l'effet à court terme de ce composé.

Les résultats sont présentés dans le tableau I :

| | | PRODUIT | | | |
|---|---|---|---|---|---|
| | | (4'-nitro.oxy-méthyl)-benzoate de 17α hydroxy-pregn-4-ène-3,20-dione | | 17α hydroxy progestérone | |
| | Concentration | Moyenne | Déviation standard | Moyenne | Déviation standard |
| Inhibition en % | 2 10-7 M | 37 | 14 | 45 | 26 |
| | 2 10-6 M | 69 | 6 | 66 | 29 |

Le dérivé nitré, (4'-nitro.oxy-méthyl)-benzoate de 17α hydroxy-pregn-4-ène-3,20-dione, possède en moyenne la même activité que l'hydroxyprogestérone, mais son action est beaucoup plus constante, moins variable.

### Exemple 5 : Inhibition in vitro de la contractilité utérine spontanée ou induite dans des utérus de rates non gestantes

Des femelles Old Wistar intactes âgées de 90 jours, pesant de 180 à 200 g, sont tuées par décapitation le lendemain du jour II du diestrous. Leur utérus entier non nettoyé est retiré et placé dans une solution de Kreb's Ringer modifiée (tampon contrôle à 2% de diméthylsulfoxide) à 37°. Le tissu est nettoyé de la graisse et du fascia qui l'enveloppent, et coupé en petits anneaux de 5 mm. Ces anneaux sont attachés à un transducteur de déplacement de force sous une tension de 1gramme. Le tissu est laissé pendant une période d'équilibration de 30 minutes afin de laisser se développer les contractions. Le tampon contrôle est remplacé par un tampon qui contient le produit à tester (exemple 1 à 3) ou la progestérone seule en présence ou non de l'antagoniste du récepteur picrotoxine (32 µg/ml) ou le mifepristone (RU-486).

Différentes concentrations du produit testé sont utilisées pour comparer les effets utéro-relaxants du produit-test aux effets de la progestérone seule. Aucun anneau de tissu n'est utilisé dans plus d'une expérience contrôle-test. La tension pour chaque type d'expérience est mesurée pendant une période de 10 mn. Les contractions utérines sont analysées en tension totale générée en gramme de tension par gramme de tissu. Cette étude a pour but de montrer l'activité utéro-relaxante du produit testé.

### Exemple 6 : Inhibition in vitro de la contractilité utérine spontanée et induit dans des utérus de cobayes gestantes

Le but est de mesurer l'action du produit-test sur des contractions utérines induites par l'oxytocine.

Des cobayes matures au 20^{e} jour de gestation sont utilisés. Un segment utérin de 10 mm est retiré et suspendu à un système de levier (1g) dans un bain organique contenant 35 ml de solution physiologique (Van Dyke-Hasting) maintenu à 37° et continuellement bullé avec O₂CO₂.

Les contractions sont induites par l'oxytocine à la concentration de 10⁻⁴ moles/ml. Les contractions sont mesurées avec un transducteur linéaire couplé à un enregistreur. L'addition du produit-test à différentes concentrations montre l'action utéro-relaxante que les contractions de l'utérus en gestation soient spontanées ou induites. L'action de ces produits est également comparée à l'action de la progestérone.

### Exemple 7 : Mesure in situ de la contractilité et de la pression artérielle dans des utérus complets

La contractilité utérine est évaluée par la mesure des changements de pression artérielle dans un utérus perfusé par une canule *in situ.* Les changements de la pression artérielle sont également déterminés. La mesure de la vitesse du sang artériel utérin reflète en effet la perfusion de cet organe.

Cinq femelles vierges de cobaye Hartley sont anesthésiées le jour 1 de leur oestrus (c'est-à-dire le premier jour de l'ouverture du vagin) avec du pentobarbital sodium à la concentration de 35 mg/kg. La veine jugulaire externe est canulée pour une administration de produit à tester et l'artère carotide est également canulée pour enregistrer la pression artérielle. L'utérus est exposé par une incision médiane abdomino-péritonéale. Une première canule est insérée à la jonction utérotubale et une deuxième canule est insérée à travers le col de l'utérus dans l'utérus et est fixée. Le liquide de perfusion (de l'eau distillée à température ambiante) est perfusé à la vitesse de 0,5 ml/mn à travers la canule de la jonction utérotubale et est collecté à partir de la canule présente dans le col de l'utérus. Le produit testé est administré et comparé à la progestérone. Les modifications de la pression de la perfusion utérine sont enregistrées avec des transducteurs de pression Beckman couplés à un enregistreur. Les réponses dans le tonus utérin sont quantifiées en termes de millimètres de pression de mercure. Les modifications de la pression artérielle sont également enregistrées.

### Exemple 8 : Mesure in vivo de la vitesse sanguine artérielle utérine et de la pression systémique dans des rates gestantes.

Des rates Wistar gestantes sont anesthésiées et ventilées de façon mécanique avec un ventilateur pour petits rongeurs. La température du corps est maintenue à 37°. La veine jugulaire est canulée pour l'injection de produits à tester et la pression systémique est enregistrée à partir de l'artère carotide gauche. L'utérus est alors exposé et maintenu humide par une solution physiologique. La rapidité artérielle utérine principale est mesurée par un Doppler.

L'administration du produit permet la mesure de l'influence des contractions utérines spontanées ou induites par la phénylephrine et également de mesurer la pression systémique. Ce test peut également être utilisé dans des études comparatives avec d'autres produits capables de relaxer l'utérus comme la progestérone seule, des donneurs de NO ou des antagonistes adrénergiques, comme par exemple la ritodrine.

### Exemple 9 : Action in vivo des produits selon l'invention sur le travail de rates gestantes.

La capacité des traitements par ces drogues à retarder le début du travail est évaluée dans un modèle qui mesure le temps de délivrance spontanée entre le premier et le second raton à naître dans une grossesse à terme.

Des femelles gestantes sont placées dans des cages individuelles en plastique à partir des jours 16 à 19 de grossesse. A partir du jour 22, les femelles sont observées de façon continue. Immédiatement à la délivrance du premier raton, les femelles reçoivent le produit à tester. Le temps de délivrance du premier raton est enregistré puis la femelle est placée dans sa cage et observée jusqu'à la délivrance du second raton. Les résultats sont exprimés en temps passé entre les première et la deuxième délivrances.

Les produits administrés sont soit le produit objet de l'invention, soit la progestérone seule, soit la ritodrine ou soit un placebo afin de comparer les effets de ces différents produits.

### Exemple 10 : Action in vivo sur la maturation cervicale utérine dans des rates gestantes.

Le but est de mesurer la maturation cervicale de rates femelles adultes. Ces rates gestantes en fin de grossesse présentant une maturation cervicale spontanée ou induite sont traitées avec le produit-test, la progestérone seule ou le placebo administrés de façon vaginale. Les mesures de temps pour terminer la délivrance, la force nécessaire pour dilater le col de l'utérus et le temps pour obtenir la dilatation sont enregistrés.

### Exemple 11: Traitement de la menace d'accouchement prématuré chez la femme

### a) par injection :

Une solution contenant un des composés des exemples 1 à 3 ou le composé 4 (dérivés de la progestérone et de la 17- hydroxyprogestérone) est reconstituée extemporanément et infusée par voie sous-cutanée, grâce à un cathéter inséré sous la peau et à l'utilisation d'une pompe à la dose initiale de 1 mg à 10 mg par heure, selon l'intensité des contractions. Cette dose peut être très progressivement augmentée si besoin sous surveillance constante de la pression artérielle. Lorsqu'une diminution de la fréquence des contractions est obtenue, le dosage peut être très progressivement diminué.

Une solution contenant un des composés des exemples 1 à 4 (dérivés de la progestérone et de la 17- hydroxyprogestérone) est injectée par voie sous-cutanée, en injection régulièrement espacées. L'utilisation des procédés habituellement utilisés en galénique peut permettre de réaliser des formes retard permettant une diffusion contrôlé, plus lente et plus prolongée du principe actif.

### b) par l'ingestion d'un comprimé

Un comprimé sublingual contenant un des composés des exemples 1 à 3 ou le composé 4 est placé sous la langue sans être avalé ni croqué. Le dosage initial est de 3 mg. La prise peut être répétée deux fois, à une heure d'intervalle (9 mg en 2 heures au total), sous surveillance continue de la pression artérielle. Les prises seront ensuite répétées toutes les 8 heures pendant une durée de 48 heures.

### c) par un gel intranasal

Un gel aqueux contenant un des composés des exemples 1 à 3 ou le composé 4, dose à 5 mg par dose unitaire, est appliqué dans une narine. Cette dose peut être répétée trois heures plus tard, sous surveillance continue de la pression artérielle. Les applications sont ensuite répétées toutes les 8 heures pendant une durée de 48 heures.

### d) par gel intravaginal

Un gel contenant un des composés des exemples 1 à 3 ou le composé 4, est inséré dans la cavité vaginale. Le dosage initial est de 10 à 200 mg de principe actif, sous surveillance continue de la pression artérielle. Une deuxième administration sera effectuée de 6 à 24 heures plus tard.

## Revendications

1. Hormone capable de se fixer à des récepteurs d'hormones, choisie parmi la progestérone, et la 170H progestérone, comportant un ou plusieurs groupements NO2 ayant au moins une des deux caractéristiques suivantes :
a) le groupe NO2 est lié directement à l'hormone par l'intermédiaire de l'atome d'oxygène de la forme énolique de l'hormone ;
b) le groupement NO2 est lié par l'intermédiaire d'un radical benzyloxy à l'hormone, soit directement à l'hormone, soit par l'intermédiaire d'un groupement établissant un pont, tel qu'un groupement C=O ou COO, à l'atome d'oxygène de la forme énolique de l'hormone ou directement sur le cycle.

2. Hormone selon la revendication 1, **caractérisée par le fait que** les récepteurs d'hormone sont choisis parmi les récepteurs stéroïdes, rétinoïdes et de dérivés de la vitamine D.

3. Hormone selon l'une quelconque des revendications 1 à 2, **caractérisée par le fait qu'**elle répond aux formules :

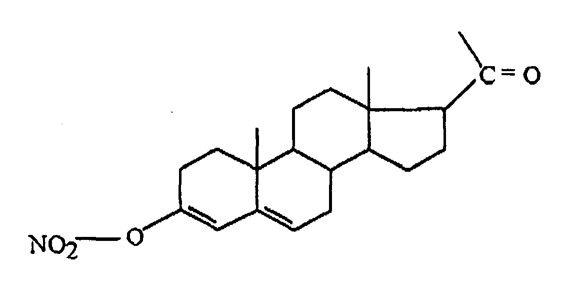

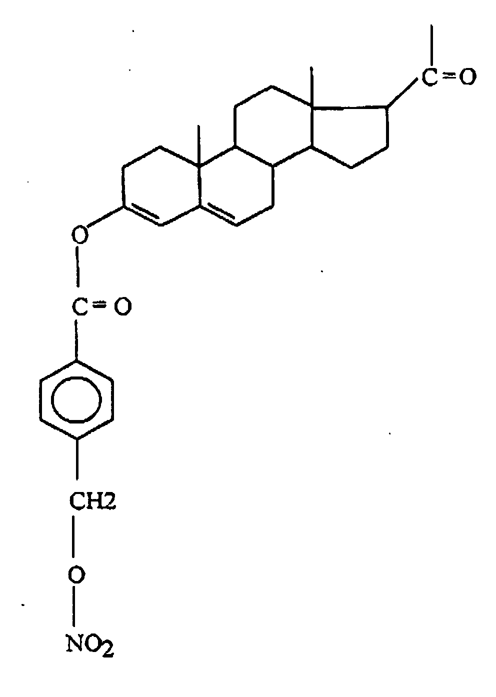

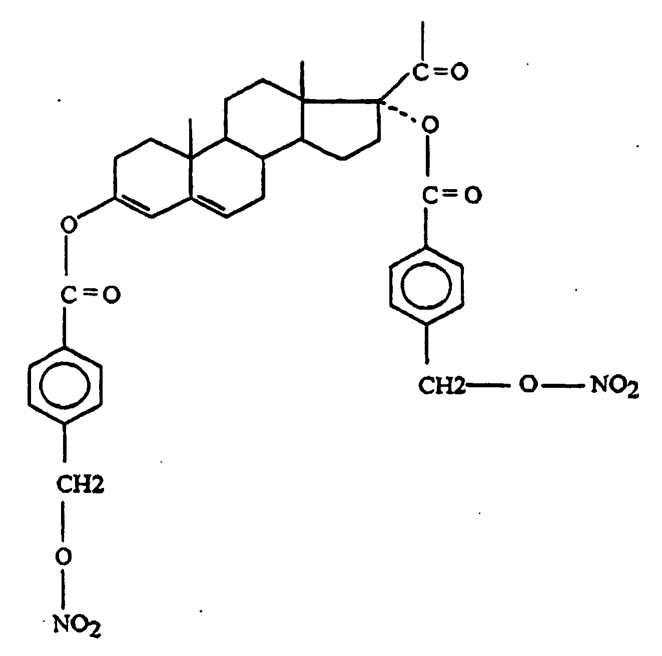

4. Hormone selon l'une quelconque des revendications 1 à 3 pour son application comme médicament

5. Composition pharmaceutique, **caractérisée par le fait qu'**elle comprend dans un milieu pharmaceutiquement acceptable, une hormone selon l'une quelconque des revendications 1 à 4.

6. Médicament selon la revendication 4 pour **son utilisation pour** la prévention des naissances prématurées.

7. Médicament selon la revendication 4 pour **son utilisation pour** le contrôle de la motilité utérine.

8. Médicament selon la revendication 4 pour **son utilisation pour** l'augmentation de la dilatation cervicale lors de l'étape du travail.

9. Médicament selon la revendication 4 pour **son utilisation pour** traiter les anomalies de la contraction utérine.

10. Médicament selon la revendication 4 pour **son utilisation pour** traiter les dysménorrhées.

11. Médicament selon la revendication 4 pour **son utilisation pour** le traitement hormonal de substitution des femmes ménopausées.

12. Médicament selon la revendication 4 pour **son utilisation pour** induire un cycle endométrial dans le cadre de la procréation assistée.

13. Médicament selon la revendication 4 pour **son utilisation pour** la prévention ou le traitement de l'hypertension artérielle.

14. Utilisation d'une hormone selon l'une des revendications 1 à 4 pour la préparation d'un médicament destiné à la prévention des naissances prématurées.

## Claims

1. Hormone capable of binding to hormone receptors, chosen from progesterone and 17OH-progesterone, comprising one or more NO₂ groups having at least one of the following two characteristics:
a) the NO₂ group is bonded directly to the hormone by means of the oxygen atom of the enolic form of the hormone;
b) the NO₂ group is bonded by means of a benzyloxy radical for the hormone, either directly to the hormone, or by means of a group that establishes a bridge, such as a C=O or COO group, to the oxygen atom of the enolic form of the hormone or directly on the ring.

2. Hormone according to Claim 1, **characterized in that** the hormone receptors are chosen from steroid receptors, retinoid receptors and vitamin D derivative receptors.

3. Hormone according to either one of Claims 1 and 2, **characterized in that** it corresponds to the formulae:

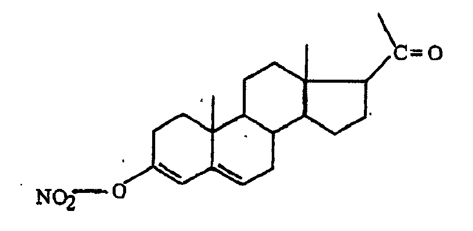

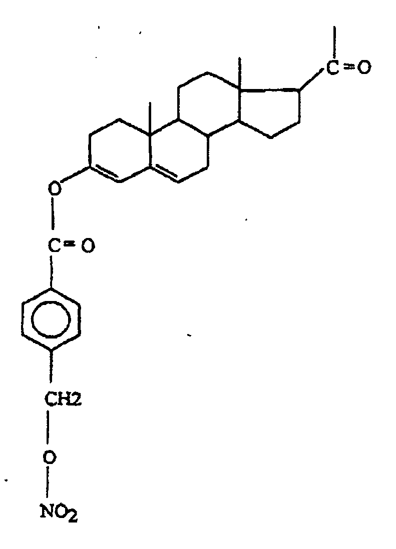

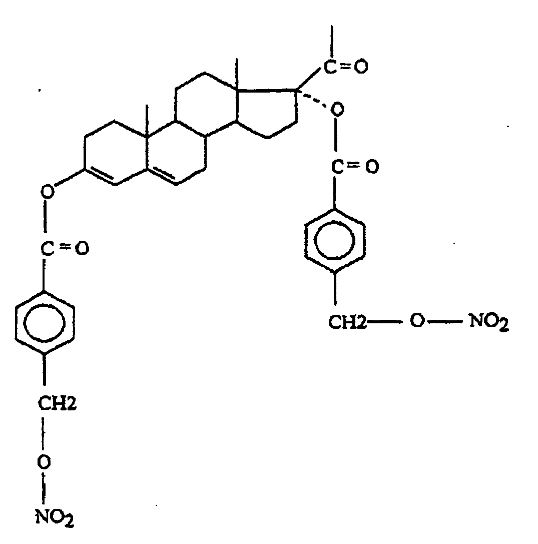

4. Hormone according to any one of Claims 1 to 3, for its use as medicinal product.

5. Pharmaceutical composition **characterized in that** it comprises, in a pharmaceutically acceptable medium, a hormone according to any one of Claims 1 to 4.

6. Medicinal product according to Claim 4, for its use for the prevention of premature births.

7. Medicinal product according to Claim 4, for its use for the control of uterine motility.

8. Medicinal product according to Claim 4, for its use for increasing cervical dilatation during labor.

9. Medicinal product according to Claim 4, for its use for treating uterine contraction anomalies.

10. Medicinal product according to Claim 4, for its use for treating dysmenorrhea.

11. Medicinal product according to Claim 4, for its use for hormone replacement therapy in menopausal women.

12. Medicinal product according to Claim 4, for its use for inducing an endometrial cycle in the context of assisted reproduction.

13. Medicinal product according to Claim 4, for its use for the prevention or treatment of arterial hypertension.

14. Use of a hormone according to one of Claims 1 to 4, for preparing a medicinal product intended for the prevention of premature births.

## Patentansprüche

1. Hormon, das zur Bindung an Hormonrezeptoren fähigt ist, unter Progesteron und 17OH-Progesteron ausgewählt ist, eine oder mehrere N02-Gruppen enthält und mindestens eines der folgenden Merkmale aufweist:
a) die N02-Gruppe ist über das Sauerstoffatom der Enol-Form des Hormons direkt an das Hormon gebunden;
b) die N02-Gruppe ist über einen Benzyloxyrest an das Hormon gebunden, entweder direkt an das Hormon oder über eine brückenbildende Gruppe, wie eine C=O- oder COO-Gruppe, an das Sauerstoffatom der Enol-Form des Hormons oder direkt an den Ring.

2. Hormon nach Anspruch 1, **dadurch gekennzeichnet, daß** die Hormonrezeptoren unter Steroidrezeptoren, Retinoidrezeptoren und Rezeptoren von Vitamin-D-Derivaten ausgewählt sind.

3. Hormon nach einem der Ansprüche 1 bis 2, **dadurch gekennzeichnet, daß** es den folgenden Formeln entspricht:

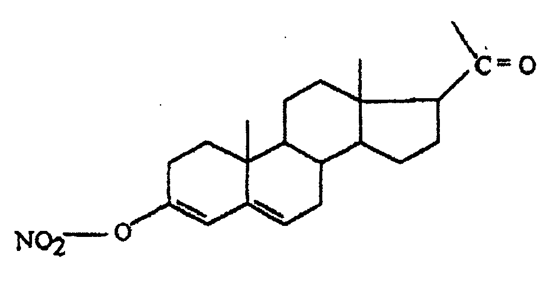

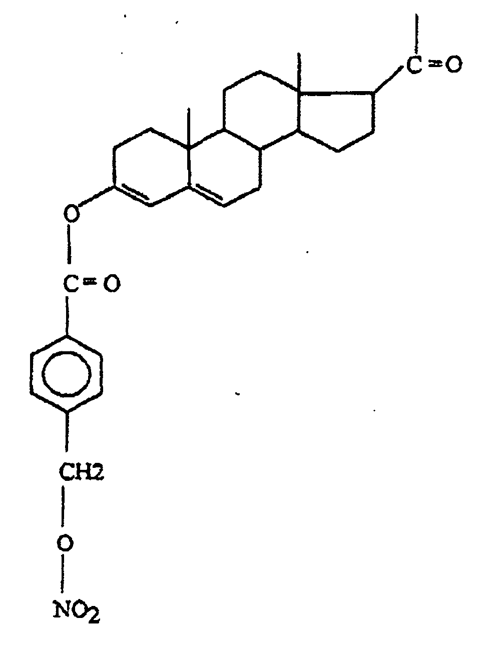

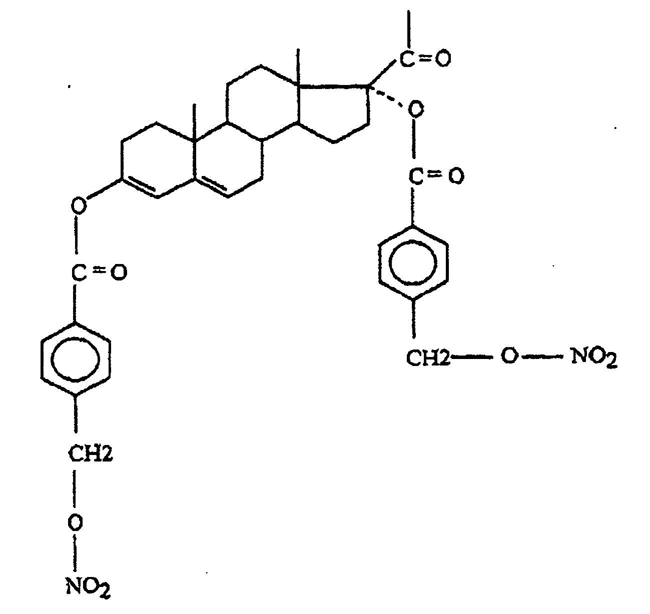

4. Hormon nach einem der Ansprüche 1 bis 3 zur Anwendung als Arzneimittel.

5. Pharmazeutische Zusammensetzung, **dadurch gekennzeichnet, daß** sie ein Hormon nach einem der Ansprüche 1 bis 4 in einem pharmazeutisch unbedenklichen Medium enthält.

6. Arzneimittel nach Anspruch 4 zur Verwendung zur Prävention von Frühgeburten.

7. Arzneimittel nach Anspruch 4 zur Verwendung zur Regulierung der Uterusmotilität.

8. Arzneimittel nach Anspruch 4 zur Verwendung zur Vergrößerung der Zervixdilatation bei den Wehen.

9. Arzneimittel nach Anspruch 4 zur Verwendung zur Behandlung von Uteruskontraktionsanomalien.

10. Arzneimittel nach Anspruch 4 zur Verwendung zur Behandlung von Dysmenorrhöen.

11. Arzneimittel nach Anspruch 4 zur Verwendung zur Hormonersatzbehandlung menopausaler Frauen.

12. Arzneimittel nach Anspruch 4 zur Verwendung zur Induktion eines endometrialen Zyklus im Rahmen der assistierten Reproduktion.

13. Arzneimittel nach Anspruch 4 zur Verwendung zur Prävention oder Behandlung von arterieller Hypertonie.

14. Verwendung eines Hormons nach einem der Ansprüche 1 bis 4 zur Herstellung eines Arzneimittels zur Prävention von Frühgeburten.
